# EUROPÄISCHE PATENTANMELDUNG

(11) **EP 3 130 284 A1**
(43) Veröffentlichungstag der Anmeldung: **15.02.2017**
(21) Anmeldenummer: 16183312.4
(22) Anmeldetag: 09.08.2016
(51) Int. Cl.: A61B 5/01, G01G 19/50, A61B 5/107

(54) **EINRICHTUNG ZUR DIAGNOSTIK VON DURCHBLUTUNGSSTÖRUNGEN UND SICH AUSBILDENDEN ENTZÜNDUNGEN BEI FÜSSEN VON PATIENTEN MIT DIABETES ODER MIT PERIPHERER ODER ZENTRALER NERVENSCHÄDIGUNG SOWIE DER OPTISCHEN DOKUMENTATION VON FUSSVERLETZUNGEN**

(30) Priorität: 10.08.2015 DE 102015215221
(71) Anmelder: Otto-von-Guericke Universität Magdeburg Medizinische Fakultät, 39120 Magdeburg (DE)
(72) Erfinder: Mertens, Peter R., 39104 Magdeburg (DE)
(74) Vertreter: Kailuweit & Uhlemann Patentanwälte Partnerschaft mbB

(57) **Zusammenfassung**

Die Erfindung betrifft Einrichtungen zur Diagnostik von Durchblutungsstörungen und sich ausbildenden Entzündungen bei Füßen von Patienten mit Diabetes oder mit peripherer oder zentraler Nervenschädigung, beispielsweise Polyneuropathie oder Schlaganfall, sowie der optischen Dokumentation von Fußverletzungen.

Die Einrichtungen zeichnen sich insbesondere dadurch aus, dass wenigstens ein Fuß des Patienten einfach erkennbar und in Folge dokumentierbar ist.

Dazu weist eine Plattform für wenigstens einen Fuß in einer Matrix angeordnete Temperatursensoren auf. Die der Seite mit der Oberfläche für den Fuß gegenüberliegende Seite der Plattform weist wenigstens eine Beleuchtungseinheit und Bildsensoren für den Fuß auf. Wenigstens ein Bereich der Plattform besteht aus einem für die Lichtstrahlen der Beleuchtungseinheit und für die vom Fuß reflektierten Lichtstrahlen transparenten Material. Die Beleuchtungseinheit, die Bildsensoren und die Temperatursensoren sind weiterhin mit einem Datenverarbeitungssystem verbunden, wobei dieses ein dem Abbild der Fußsohle Temperaturen zuordnendes Datenverarbeitungssystem ist.

## Beschreibung

Die Erfindung betrifft Einrichtungen zur Diagnostik von Durchblutungsstörungen und sich ausbildenden Entzündungen bei Füßen von Patienten mit Diabetes oder mit peripherer oder zentraler Nervenschädigung, beispielsweise Polyneuropathie oder Schlaganfall, sowie der optischen Dokumentation von Fußverletzungen.

Eine Folgewirkung von Diabetes ist ein Gefühlsverlust insbesondere im Fuß. Dadurch können Druckstellen entstehen, die zu Wunden führen, ohne dass dies der Patient merkt. Zur Vorbeugung und Kontrolle sind verschiedene Einrichtungen bekannt, um einer schädigenden Druckbelastung vorzubeugen.

Durch die Druckschrift DE 92 03 788 U1 ist eine Schuheinlage zur Ulcusprohylaxe am diabetischen Fuß bekannt. Dazu ist in einer Einlegesohle, welche sich ganz oder größtenteils entlang der Fußsohle erstreckt, im Bereich des Vorfußes eine kompressible Hydrozelle aus flexiblem Kunststoffmaterial angeordnet. Weiterhin steht ein Sensor zur Druckerfassung mit der Flüssigkeit in der Hydrozelle in Verbindung.

Die Druckschrift US 4,647,918 A beschreibt eine Einrichtung zur Erfassung der Druckbelastung an mehreren Stellen des Fußes. Dazu wird eine Schuheinlage mit mehreren Drucksensoren verwendet. Die Drucksensoren sind an verschiedenen Stellen des Fußes platziert. Eine Signalisierung erfolgt mittels eines Geräts, welches zum Einen mit den Sensoren verbunden und zum Anderen durch den Patienten tragbar ist. Die Auswertung erfolgt zeitbezogen über einen Mikroprozessor, der über einen Kabelbaum elektrisch leitend mit den Messwertgebern verbunden ist, entsprechend vorgegebener und gespeicherter Grenzwerte.

Durch die Druckschrift 10 2010 063 740 A1 ist eine Schutzeinrichtung für den diabetischen Fuß bekannt, die im Wesentlichen aus einer Sohle mit Seitenteilen, Sensoren als Druck- und Temperatursensoren, einem Datenverarbeitungssystem, einem Sender, einer Energiequelle, einem Empfänger und einem Signalgeber besteht. Diese Schutzeinrichtung bezieht sich insbesondere auf die Seitenteile mit Druck- und Temperatursensoren zur Erfassung von Eigenschaften der seitlichen Bereiche der Füße.

Mittels Temperatur- und/oder Druckmessungen wird auf die Eigenschaften des Fußes geschlossen. Eine optische Erfassung ist dabei nicht vorgesehen.

Der im Patentanspruch 1 angegebenen Erfindung liegt die Aufgabe zugrunde, den diabetischen Fuß eines Patienten oder einen Fuß bei anderen Erkrankungen mit Empfindungsstörungen einfach zu erkennen und zu dokumentieren.

Diese Aufgabe wird mit den im Patentanspruch 1 aufgeführten Merkmalen gelöst.

Die Einrichtungen zur Diagnostik von Durchblutungsstörungen und sich ausbildenden Entzündungen bei Füßen von Patienten mit Diabetes oder mit peripherer oder zentraler Nervenschädigung, beispielsweise Polyneuropathie oder Schlaganfall, sowie der optischen Dokumentation von Fußverletzungen zeichnen sich insbesondere dadurch aus, dass wenigstens ein Fuß des Patienten einfach erkennbar und in Folge dokumentierbar ist.

Dazu weist eine Plattform für wenigstens einen Fuß in einer Matrix angeordnete Temperatursensoren auf. Die der Seite mit der Oberfläche für den Fuß gegenüberliegende Seite der Plattform weist wenigstens eine Beleuchtungseinheit und Bildsensoren für den Fuß auf. Wenigstens ein Bereich der Plattform besteht aus einem für die Lichtstrahlen der Beleuchtungseinheit und für die vom Fuß reflektierten Lichtstrahlen transparenten Material. Die Beleuchtungseinheit, die Bildsensoren und die Temperatursensoren sind weiterhin mit einem Datenverarbeitungssystem verbunden, wobei dieses ein dem Abbild der Fußsohle Temperaturen zuordnendes Datenverarbeitungssystem ist.

Eine wesentliche Maßnahme zur Prävention bei Diabetikern mit Polyneuropathie und/oder Makroangiopathie und drohendem "Diabetischem Fußsyndrom" oder drohendem Charcot-Fuß ist die regelmäßige optische Inspektion. Diese wird jedoch aufgrund der körperlichen Einschränkungen der Patienten oft nur sporadisch und indirekt mit Zuhilfenahme von Spiegeln durchgeführt. Die in dieser Personengruppe oftmals geringe Adhärenz beim Einsatz verfügbarer Einlegesohlen (passiv oder mit Messeinrichtung) trägt oftmals zu einer Verschlechterung bei und verhindert eine kontinuierliche Beurteilung des Fußzustandes.

Regelmäßige Temperaturmessungen des Fußes sind hilfreich und in der Primär- sowie Sekundärprävention vorteilhaft. Die erfindungsgemäße Einrichtung dient der regelmäßigen

Dokumentation des Fußzustandes durch Akquise von Bildern sowie Informationen der dabei vorherrschenden Temperaturen. Damit ist eine zeitnahe Erkennung einer Fußgeschwürentwicklung möglich. Die parallele Dokumentation des Fußes mittels optischer Verfahren gestattet dem Patienten eine Eigenbeurteilung und dient dem Arzt als zusätzliches Diagnosehilfsmittel. Der Patient kann natürlich auch eine Patientin sein. Dazu kann der Patient die Plattform einfach betreten. Dazu kann der Patient einen Fuß auf die Plattform platzieren oder sich mit beiden Füßen auf die Plattform stellen. Die Temperatursensoren messen gleichzeitig die Fußtemperatur an allen Temperatursensoren, die mit der Fußsohle des Patienten in Kontakt kommen. Mittels der Beleuchtungseinheit und den Bildsensoren für den Fuß wird das Abbild des Fußes oder die Abbilder der Füße gewonnen. Mittels des Datenverarbeitungssystems wird wenigstens ein Abbild den Temperaturen zugeordnet, wobei die jeweiligen Positionen durch die Positionen der Temperatursensoren in der Matrix bestimmt sind. Damit ist eine eindeutige Zuordnung gegeben. Zur Dokumentation werden die Daten im Datenverarbeitungssystem gespeichert, wobei den Daten die Zeit und der Patient zugeordnet werden. Weiterhin generiert das Datenverarbeitungssystem aus den gemessenen Temperaturwerten mittels eines Algorithmus die Verdachtsdiagnose, insbesondere hinsichtlich eines drohendes Geschwürs oder einer Minderdurchblutung.

Damit eignet sich die erfindungsgemäße Einrichtung vorteilhafterweise zur
- Früherkennung und Frühwarnung eines sich bildenden Geschwürs, einer Fußdeformität und einer Verletzung an einer oder mehrerer Stellen am Fuß,
- Erkennung von Langzeitveränderungen im Temperaturprofil eines Fußes, beispielsweise aufgrund von Änderungen der Durchblutung, und
- optischen Dokumentation des Fußzustandes.

Ein wesentlicher Vorteil ist damit die Kombination aus Dokumentation der Abbilder und Temperaturmessung. Damit ist eine frühzeitige Prävention einer Geschwürbildung möglich.

Eine mögliche Geschwürbildung kann durch die erfindungsgemäße Einrichtung durch Messung eines Temperaturanstiegs im Falle einer entstehenden Entzündung erkannt werden. Vor Ausbruch eines Fußgeschwürs steigt die Temperatur etwa eine Woche im Voraus lokal an. Im Vergleich mit den Vortemperaturwerten und den anderen Messwerten des gleichen oder des anderen Fußes können Rückschlüsse auf ein beginnendes Ulkus gezogen werden. An einem oder mehreren Sensoren kann dazu ein relativer Anstieg der Temperatur durch das Datenverarbeitungssystem im Vergleich zu den identischen Messpunkten der Sohle des zweiten Fußes erkannt werden. Dazu werden die Temperaturen beider Füße nacheinander gemessen. Weiterhin erfolgt durch die Bildsensoren eine Fußabbildung sowie eine Umrissdetektion. Diese dienen der wiederholungssicheren Positionierung der Sensorpunkte innerhalb des Koordinatensystems der Fußsohle. Der Patient kann über das Datenverarbeitungssystem durch optische oder akustische Signale durch die Messphasen geleitet werden. Überschreitet der Differenzwert zwischen den Messpunkten beider Füße einen Grenzwert, kann eine Warninformation über die optischen und akustischen Signalisierungseinrichtungen abgegeben werden. Gleichzeitig kann ein Warnsignal über einen mit dem Datenverarbeitungssystem verbundenen Sender an einen Empfänger drahtlos gesendet. Der Patient erkennt dadurch, dass die Gefahr besteht, dass sich ein Ulkus bildet. Weiterhin wird dieser zur Kontrolle der durch die erfindungsgemäße Einrichtung aufgenommenen bildlichen Darstellung des Fußes auf lokale Veränderungen, wie Rötung, Überwärmung, Hautveränderungen, aufgefordert und darauf hingewiesen, dass ein Arzt aufgesucht werden sollte. Die Anzeige kann zusätzlich durch mobile Applikationen erfolgen. Gleichzeitig können die Signale und/oder Daten, wie beispielsweise das Temperaturprofil und die bildlichen Darstellung der Füße, über ein Datennetz an ein zentrales Computerauswertungssystem gesendet werden.

Dieses System verarbeitet die empfangenen Informationen und sendet sie weiter an die Ärztesoftware. Der Arzt wird somit ebenfalls von dem entstehenden Fußgeschwür seines Patienten in Kenntnis gesetzt und kann gemeinsam mit dem Patienten Präventivmaßnahmen einleiten.

Bei einer regionalen Minderdurchblutung in Fußbereichen oder des gesamten Fußes bei einer Durchblutungsstörung fällt die Temperatur ab. Die Minderdurchblutung kann akut oder chronisch eintreten, oftmals stellt sie einen wichtigen prädisponierenden Faktor für eine critical limb ischemia dar.

Durch die Langzeitbeobachtung des Temperaturprofils innerhalb eines Fußes kann eine solche Minderdurchblutung erkannt werden. Dies kann dem Patienten und Arzt in vergleichbarer Form, wie bei der möglichen Geschwürbildung beschrieben, signalisiert werden. Der Arzt kann die Diagnose durch Zusatzuntersuchungen (Arm-Bein-Index/Ankle-Brachial-Index, Angiographie) bestätigen und eine medikamentöse Therapie einleiten oder eine Gefäßbehandlung durchführen.

Bei einem Charcot-Fuß kommt es zu Entzündungsreaktionen nach Frakturen und lokaler Überwärmung. Die Erkennung eines Charcotfußes im Frühstadium ist durch regelmäßige Temperaturmessungen möglich. Zudem kann eine Hyperämie durch den Fußscan gesichtet werden. Weiterhin ist eine Therapieverlaufsuntersuchung durch die Temperaturmessung möglich.

Die Bildsensoren generieren eine fotorealistische Abbildung der Füße. Dieses wird in dem Datenverarbeitungssystem gespeichert. Die Information kann über den Sender weitergeleitet werden. Eine Anzeige kann über einen Bildschirm oder die mobile Applikation erfolgen. Die Übertragung an den Arzt kann vergleichbar der möglichen Geschwürbildung erfolgen.

Damit eignet sich die erfindungsgemäße Einrichtung zur Prävention von Ulzerationen bei bestehender (Poly-)Neuropathie oder bei Durchblutungsstörungen im Rahmen von Diabetes und anderen Erkrankungen mit einhergehender Nervenschädigung und/oder Minderdurchblutung.

Vorteilhafte Ausgestaltungen der Erfindung sind in den Patentansprüchen 2 bis 13 angegeben.

Die Beleuchtungseinheit und die Bildsensoren sind nach der Weiterbildung des Patentanspruchs 2 Bestandteile einer Scaneinheit, die eine optomechanische Vorrichtung ist. Wenigstens eine Fußsohle wird beleuchtet und das davon reflektierte Licht gelangt an die in einer Zeile angeordneten Bildsensoren als optoelektronischer Zeilensensor. Die analogen Lichtsignale werden in Digitalsignale gewandelt, aus denen im Datenverarbeitungssystem das Abbild generiert wird.

Die Plattform ist nach der Weiterbildung des Patentanspruchs 3 eine Plattform für die Füße eines Patienten. Weiterhin weist die der Seite mit der Oberfläche für die Füße gegenüberliegende Seite der Plattform beabstandet zueinander zwei Scaneinheiten für die Füße des Patienten. Der Patient stellt sich hierbei auf die Plattform und beide Füße werden abgescannt.

Das Datenverarbeitungssystem ist nach der Weiterbildung des Patentanspruchs 4 ein
- aus den Temperaturen der in der Matrix angeordneten Temperatursensoren ein das Temperaturprofil des Fußes ermittelndes
   und
- das aus den Temperaturen ermittelte Profil dem Abbild der Bildsensoren zuordnendes oder
- dem Abbild der Bildsensoren das aus den Temperaturen ermittelte Profil zuordnendes Datenverarbeitungssystem. Mittels des Datenverarbeitungssystems wird damit ein Abbild des Fußes mit zugehörigen Temperaturen ermittelt. Grundlage dazu kann insbesondere der Umriss des Fußes sein, der sowohl über die Temperatursensoren als auch über das Abbild gewonnen werden kann. Bei letzterem können dazu bekannte Verfahren einer Kantendetektion verwendet werden. Die Umrisse werden übereinander projiziert. Darüber hinaus können diese Messwerte auch zu einer Signalisierung genutzt werden, wobei bei Über- oder Unterschreiten einer Temperaturschwelle ein optisches und/oder akustisches Warnsignal abgegeben werden kann.

Das Datenverarbeitungssystem ist nach der Weiterbildung des Patentanspruchs 5 ein wenigstens einem Bereich mit gegenüber der Temperatur der Fußsohle erhöhter oder verminderter Temperatur einen gegenüber der Farbe des Abbildes der Fußsohle andere Farbe zuordnendes Datenverarbeitungssystem. Damit sind Bereiche unterschiedlicher Temperaturen darstellbar, so dass eine Minderdurchblutung bei einer verminderter Temperatur und/oder eine Geschwürbildung bei erhöhter Temperatur optisch dargestellt werden kann.

Das Datenverarbeitungssystem ist nach der Weiterbildung des Patentanspruchs 6 ein den Temperaturen der Fußsohle gegenüber der Farbe des Abbildes der Fußsohle andere Farben zuordnendes Datenverarbeitungssystem, wobei gleichen Temperaturen gleiche Farben zugeordnet werden. Gebiete mit einer Minderdurchblutung oder einer Gefahr der Ausbildung von Geschwüren können damit besonders hervorgehoben werden. Das kann auch mit einer Falschfarbendarstellung erfolgen, so dass auch schwache Kontraste erkennbar sind.

Die in einer Matrix angeordneten Temperatursensoren und die Scaneinheit sind nach der Weiterbildung des Patentanspruchs 7 entweder nebeneinander angeordnet oder die Plattform und die Temperatursensoren bestehen aus einem für die Lichtstrahlen der Beleuchtungseinheit transparenten Material und die in einer Matrix angeordneten Temperatursensoren und die Scaneinheit sind übereinander angeordnet.

Das Datenverarbeitungssystem ist nach der Weiterbildung des Patentanspruchs 8
- ein aus den Temperaturen die Position und/oder die Abmessungen des Fußes und die Position und Abmessung des wenigstens eines Bereichs erhöhter oder verringerter Temperatur oder
- ein die Positionen und Abmessungen der Bereiche unterschiedlicher Temperatur ermittelndes und dem Abbild der Fußsohle zuordnendes Datenverarbeitungssystem.

Das Datenverarbeitungssystem ist nach der Weiterbildung des Patentanspruchs 9 ein dem Abbild des Fußes ein Koordinatensystem zuordnendes Datenverarbeitungssystem. Veränderungen des Fußes hinsichtlich seiner Geometrie sind leicht nachvollziehbar.

Die Beleuchtungseinheit ist nach der Weiterbildung des Patentanspruchs 10 eine sichtbares und/oder infrarotes Licht sendende Beleuchtungseinheit.

Das Datenverarbeitungssystem ist nach der Weiterbildung des Patentanspruchs 11 mit wenigstens einem Bildschirm zur Darstellung wenigstens eines Abbildes einer Fußsohle oder zur Darstellung wenigstens eines Abbildes einer Fußsohle und wenigstens eines älteren Abbildes der gespeicherten Fußsohle zusammengeschaltet. Entwicklungen des Fußes können damit leicht überprüft werden. Verschlechterungen oder Verbesserungen des Krankheitsbildes sind einfach zu ermitteln. Das Wirken insbesondere von Medikamenten ist leicht kontrollierbar.

Das Datenverarbeitungssystem ist nach der Weiterbildung des Patentanspruchs 12 mit einem Sender/Empfänger für elektromagnetische Strahlung zur Datenübertragung an wenigstens einen mit einem weiteren Datenverarbeitungssystem verbundenen Sender/Empfänger verbunden. Damit können insbesondere Warnsignale und weitere Informationen an einen externen Empfänger beispielsweise bei einem Arzt übertragen werden. Darüber hinaus kann der Empfänger gleichzeitig Informationen von externen Geräten empfangen, so dass eine direkte Kommunikation mit dem Arzt gegeben ist. Natürlich können die übertragenen Daten auch gespeichert werden, so dass sich der Arzt jederzeit über den Zustand des Patienten informieren kann.

Die Plattform ist nach der Weiterbildung des Patentanspruchs 13 ein Bestandteil einer Personenwaage, wobei die Einrichtung zur Messwertaufnahme mit dem Datenverarbeitungssystem zur Ermittlung und Speicherung des Gewichts und/oder Körpermasseindex des Patienten verbunden ist.

Ein Ausführungsbeispiel der Erfindung ist in den Zeichnungen jeweils prinzipiell dargestellt und wird im Folgenden näher beschrieben.

Es zeigen:
- Fig. 1: eine Einrichtung zur Diagnostik von Durchblutungsstörungen und sich ausbildenden Entzündungen bei Füßen von Patienten mit Diabetes sowie der optischen Dokumentation von Fußverletzungen mit zwei Bereichen zum Bildscan und einem Bereich mit Temperatursensoren in einer Draufsicht,
- Fig. 2: eine Einrichtung in einer Seitenansicht,
- Fig. 3: eine Einrichtung mit einem Bereich zum Bildscan und einem Bereich mit Temperatursensoren in einer Draufsicht und
- Fig. 4: eine Einrichtung mit zwei Bereichen mit Temperatursensoren und zum Bildscan in einer Draufsicht.

Eine Einrichtung zur Diagnostik von Durchblutungsstörungen und sich ausbildenden Entzündungen bei Füßen von Patienten mit Diabetes sowie der optischen Dokumentation von Fußverletzungen besteht im Wesentlichen aus einer Plattform 1, Temperatursensoren 2, einer Scaneinheit 5 und einem Datenverarbeitungssystem.

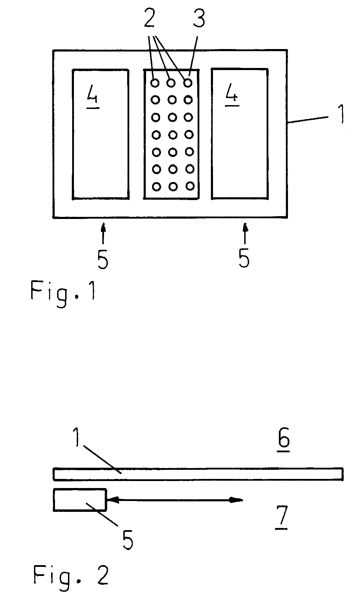

Die Fig. 1 zeigt eine Fig. 1 eine Einrichtung zur Diagnostik von Durchblutungsstörungen und sich ausbildenden Entzündungen bei Füßen von Patienten mit Diabetes sowie der optischen Dokumentation von Fußverletzungen mit zwei Bereichen zum Bildscan und einem Bereich mit Temperatursensoren in einer prinzipiellen Draufsicht.

Die Plattform 1 ist zum Betreten des Patienten ausgelegt und besitzt drei Bereiche 3, 4. Ein Bereich 3 weist die in einer Matrix angeordneten Temperatursensoren 2 auf. Dieser Bereich 3 ist zwischen zwei Bereichen 4 zum Bildscan angeordnet.

Die Fig. 2 zeigt eine Einrichtung in einer prinzipiellen Seitenansicht.

Die der Seite 6 mit der Oberfläche für den Fuß gegenüberliegende Seite 7 der Plattform 1 weist die Scaneinheiten 5 auf, die jeweils wenigstens eine Beleuchtungseinheit und Bildsensoren für den Fuß umfassen. Wenigstens die Bereiche 4 der Plattform 1 bestehen aus einem für die Lichtstrahlen der Beleuchtungseinheit und für die vom Fuß reflektierten Lichtstrahlen transparenten Material. Die Scaneinheiten 5 und die Temperatursensoren 2 sind mit dem Datenverarbeitungssystem verbunden. Das kann ein Mikrorechner an der Einrichtung oder wenigstens ein externer Computer sein. Die Verbindungen können drahtgebunden und/oder drahtlos ausgebildet sein, wobei bei letzteren ein Sender/Empfänger für elektromagnetische Strahlung zur Datenübertragung mit dem Datenverarbeitungssystem verbunden ist. Die Scaneinheit 5 besitzt sowohl die Beleuchtungseinheit und die in einer Reihe angeordneten Bildsensoren zum Empfang der von der Fußsohle reflektierten Lichtstrahlen. Für die Gewinnung der Daten der Fußsohle sind die Beleuchtungseinheit und die Bildsensoren geführt verfahrbar angeordnet und an einen Antrieb gekoppelt, wobei diese Teile im Wesentlichen die Scaneinheit 5 sind, deren Realisierung bekannt ist.

Der Patient stellt sich auf die Plattform 1 mit den Füßen auf die Bereiche 4 zum Bildscan. Die Scaneinheiten 5 scannen eine oder die Fußsohlen zur Ermittlung dessen oder deren Abbilder. Danach stellt der Patient einen Fuß oder nacheinander die Füße auf den Bereich 3 mit den Temperatursensoren 2.

Das Datenverarbeitungssystem ist ein
- aus den Temperaturen das Profil des Fußes ermittelndes,
- die Temperaturen in Abhängigkeit der Positionen der Temperatursensoren 2 dem Profil des Fußes zuordnendes und
- entweder das aus den Temperaturen ermittelte Profil dem Abbild der Bildsensoren der Scaneinheit 5 zuordnendes
- oder dem Abbild der Bildsensoren der Scaneinheit 5 das aus den Temperaturen ermittelte Profil zuordnendes Datenverarbeitungssystem ist.

Damit besitzt die Einrichtung zwei Scanfunktionen, zum einen einen Bildscan zur Bilddokumentation mittels der Scaneinheit 5 und zum anderen einen Scan zur Bestimmung des Fußes mittels der Temperatursensoren 2. Durch Superposition der Fußumrisse und der Bilddokumentation nach direkter Bilddokumentation wird das Abbild des Fußes im Datenverarbeitungssystem generiert, dem gleichzeitig die Temperaturen entsprechend der Position der Temperatursensoren 2 zugeordnet werden. Damit ist das Datenverarbeitungssystem ein aus den Temperaturen die Position und/oder die Abmessungen des Fußes und
- entweder die Position und Abmessung des wenigstens eines Bereichs erhöhter oder verringerter Temperatur
- oder die Positionen und Abmessungen der Bereiche unterschiedlicher Temperatur ermittelndes und dem Abbild der Fußsohle und damit des Fußes zuordnendes Datenverarbeitungssystem.
Diesen Bereichen können bestimmte Farben auch als Falschfarben zugeordnet werden.

Das Datenverarbeitungssystem und/oder ein weiteres Datenverarbeitungssystem kann mit wenigstens einem Bildschirm zur Darstellung wenigstens eines Abbildes einer Fußsohle oder zur Darstellung wenigstens eines Abbildes einer Fußsohle und wenigstens eines älteren Abbildes der gespeicherten Fußsohle zusammengeschaltet sein. Dazu kann dieses mit einem Sender/Empfänger für elektromagnetische Strahlung zur Datenübertragung an wenigstens einen mit einem weiteren Datenverarbeitungssystem verbundenen Sender/Empfänger verbunden sein.

Zusätzlich kann die Plattform 1 ein Bestandteil einer Personenwaage sein. Die die Einrichtung zur Messwertaufnahme ist dazu mit dem Datenverarbeitungssystem zur Ermittlung und Speicherung des Gewichts des Patienten verbunden.

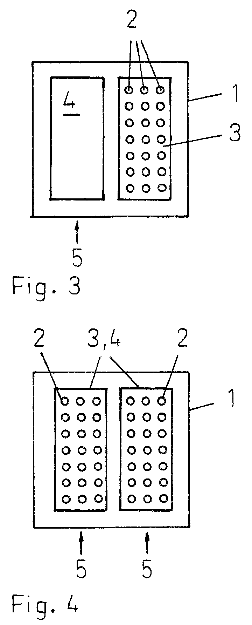

Die Fig. 3 zeigt eine Einrichtung mit einem Bereich 4 zum Bildscan und einem Bereich 3 mit Temperatursensoren 2 in einer prinzipiellen Draufsicht.

In einer Ausführungsform kann eine Einrichtung zur Erkennung und Dokumentation des diabetischen Fußes eines Patienten oder des Fußes eines Patienten mit einer Nervenschädigung aufgrund anderer Erkrankungen im Wesentlichen auch aus einer Plattform 1 mit jeweils einem Bereich 3, 4 mit Temperatursensoren 2 und einer Scaneinheit 5 bestehen. Damit können die Füße gescannt werden, wobei das ein Fuß, der nacheinander gescannt wird, oder nach Drehen des Patienten auf der Plattform 1 beide Füße sein können.

Die Fig. 4 zeigt eine Einrichtung mit zwei Bereichen 3 mit Temperatursensoren 2 und zum Bildscan in einer Draufsicht.

In einer weiteren Ausführungsform können die Temperatursensoren 2 aus einem für die Lichtstrahlen transparenten Material bestehen. Damit können beide Füße gleichzeitig gescannt werden, wobei dabei sowohl ein Bildscan als auch ein Temperaturscan erfolgt. Die Beleuchtungseinheit kann dabei eine sichtbares und/oder infrarotes Licht sendende Beleuchtungseinheit sein. Die Bildsensoren können bei infraroten Licht die Bestandteile einer Infrarotkamera sein.

### Bezugszeichenliste

- 1: Plattform
- 2: Temperatursensor
- 3: Bereich mit Temperatursensoren
- 4: Bereich zum Bildscan
- 5: Scaneinheit
- 6: Seite für Fuß
- 7: Seite mit Scaneinheit

## Patentansprüche

1. Einrichtung zur Diagnostik von Durchblutungsstörungen und sich ausbildenden Entzündungen bei Füßen von Patienten mit Diabetes oder peripherer oder zentraler Nervenschädigung sowie der optischen Dokumentation von Fußverletzungen, **dadurch gekennzeichnet, dass** eine Plattform (1) für wenigstens einen Fuß in einer Matrix angeordnete Temperatursensoren (2) aufweist, dass die der Seite (6) mit der Oberfläche für den Fuß gegenüberliegende Seite (7) der Plattform (1) wenigstens eine Beleuchtungseinheit und Bildsensoren für den Fuß aufweist, dass wenigstens ein Bereich der Plattform (1) aus einem für die Lichtstrahlen der Beleuchtungseinheit und für die vom Fuß reflektierten Lichtstrahlen transparenten Material besteht, dass die Beleuchtungseinheit, die Bildsensoren und die Temperatursensoren (2) mit einem Datenverarbeitungssystem verbunden sind und dass das Datenverarbeitungssystem ein dem Abbild der Fußsohle Temperaturen zuordnendes Datenverarbeitungssystem ist.

2. Einrichtung nach Patentanspruch 1, **dadurch gekennzeichnet, dass** die Beleuchtungseinheit und Bildsensoren Bestandteile einer Scaneinheit (5) sind.

3. Einrichtung nach den Patentansprüchen 1 und 2, **dadurch gekennzeichnet, dass** die Plattform (1) eine Plattform (1) für die Füße eines Patienten ist und dass die der Seite (6) mit der Oberfläche für die Füße gegenüberliegende Seite (7) der Plattform (1) beabstandet zueinander zwei Scaneinheiten (5) für die Füße des Patienten aufweist.

4. Einrichtung nach Patentanspruch 1, **dadurch gekennzeichnet, dass** das Datenverarbeitungssystem ein
- aus den Temperaturen der in der Matrix angeordneten Temperatursensoren (2) ein das Temperaturprofil des Fußes ermittelndes und
- das aus den Temperaturen ermittelte Profil dem Abbild der Bildsensoren zuordnendes oder
- dem Abbild der Bildsensoren das aus den Temperaturen ermittelte Profil zuordnendes Datenverarbeitungssystem ist.

5. Einrichtung nach den Patentansprüchen 1 und 4, **dadurch gekennzeichnet, dass** das Datenverarbeitungssystem ein wenigstens einem Bereich mit gegenüber der Temperatur der Fußsohle erhöhter oder verminderter Temperatur einen gegenüber der Farbe des Abbildes der Fußsohle andere Farbe zuordnendes Datenverarbeitungssystem ist.

6. Einrichtung nach den Patentansprüchen 1 und 4, **dadurch gekennzeichnet, dass** das Datenverarbeitungssystem ein den Temperaturen der Fußsohle gegenüber der Farbe des Abbildes der Fußsohle andere Farben zuordnendes Datenverarbeitungssystem ist, wobei gleichen Temperaturen gleiche Farben zugeordnet werden.

7. Einrichtung nach den Patentansprüchen 1 und 2, **dadurch gekennzeichnet, dass** die in einer Matrix angeordneten Temperatursensoren (2) und die Scaneinheit (5) nebeneinander angeordnet sind oder dass die Plattform (1) und die Temperatursensoren (2) aus einem für die Lichtstrahlen der Beleuchtungseinheit transparenten Material bestehen und dass die in einer Matrix angeordneten Temperatursensoren (2) und die Scaneinheit (5) übereinander angeordnet sind.

8. Einrichtung nach den Patentansprüchen 1 und 2 oder 1, 2 und 7, **dadurch gekennzeichnet, dass** das Datenverarbeitungssystem ein aus den Temperaturen die Position und/oder die Abmessungen des Fußes und die Position und Abmessung des wenigstens eines Bereichs erhöhter oder verringerter Temperatur oder die Positionen und Abmessungen der Bereiche unterschiedlicher Temperatur ermittelndes und dem Abbild der Fußsohle zuordnendes Datenverarbeitungssystem ist.

9. Einrichtung nach Patentanspruch 1, **dadurch gekennzeichnet, dass** das Datenverarbeitungssystem ein dem Abbild des Fußes ein Koordinatensystem zuordnendes Datenverarbeitungssystem ist.

10. Einrichtung nach Patentanspruch 1, **dadurch gekennzeichnet, dass** die Beleuchtungseinheit eine sichtbares und/oder infrarotes Licht sendende Beleuchtungseinheit ist.

11. Einrichtung nach Patentanspruch 1, **dadurch gekennzeichnet, dass** das Datenverarbeitungssystem mit wenigstens einem Bildschirm zur Darstellung wenigstens eines Abbildes einer Fußsohle oder zur Darstellung wenigstens eines Abbildes einer Fußsohle und wenigstens eines älteren Abbildes der gespeicherten Fußsohle zusammengeschaltet ist.

12. Einrichtung nach Patentanspruch 1, **dadurch gekennzeichnet, dass** das Datenverarbeitungssystem mit einem Sender/Empfänger für elektromagnetische Strahlung zur Datenübertragung an wenigstens einen mit einem weiteren Datenverarbeitungssystem verbundenen Sender/Empfänger verbunden ist.

13. Einrichtung nach Patentanspruch 1, **dadurch gekennzeichnet, dass** die Plattform (1) ein Bestandteil einer Personenwaage ist und dass die Einrichtung zur Messwertaufnahme mit dem Datenverarbeitungssystem zur Ermittlung und Speicherung des Gewichts und/oder Körpermasseindex des Patienten verbunden ist.
